Europäisches Patentamt

(19) European Patent Office

Office européen des brevets

(11) Publication number: **0 030 977**
**B1**

# EUROPEAN PATENT SPECIFICATION

(45) Date of publication of patent specification: **25.07.84**

(51) Int. Cl.³: **A 61 K 35/78**

(21) Application number: **80900424.5**

(22) Date of filing: **19.02.80**

(86) International application number:
**PCT/JP80/00022**

(87) International publication number:
**WO 80/02503 27.11.80 Gazette 80/27**

(54) **PROPHYLACTIC AND CURATIVE MEDICINE FOR CIRRHOTIC LIVER.**

(30) Priority: **14.05.79 JP 58119/79**

(43) Date of publication of application:
**01.07.81 Bulletin 81/26**

(45) Publication of the grant of the patent:
**25.07.84 Bulletin 84/30**

(84) Designated Contracting States:
**FR**

(56) References cited:

**CHEMICAL ABSTRACTS, vol. 73. no. 3, July 20, 1970, page 216, abstract 13031t, COLUMBUS OHIO (US), K.Y. YEN et al.: "Pharmacological effective components of local plants in Taiwan. II. Antibiotic activity and pharmacological action of Ampelopsis brevipedunculata varhancei" Journal of the Taiwan Pharmaceutical Association, vol. 19, no. 1, p. 15-19 (1967)**

The file contains technical information submitted after the application was filed and not included in this specification

(73) Proprietor: **EDUCATIONAL FOUNDATION DOKKYO GAKUEN**
**8-1, Sekigu-chi 3-chome**
**Bunkyo-ku Tokyo 112 (JP)**

(72) Inventor: **KATSUTA, Hajim Jap. Res. Center of Tissue Culture**
**Dokkyo University 880 Kita-Kobayashi Mibu-machi Shimo-tsuga-gun Tochigi321-02 (JP)**
Inventor: **SEKI, Minato Jap. Res. Center of Tissue Culture**
**Dokkyo University 880 Kita-Kobayashi Mibu-machi Shimo-tsuga-gun Tochigi321-02 (JP)**

(74) Representative: **Bressand, Georges et al, c/o CABINET LAVOIX 2 Place d'Estienne d'Orves F-75441 Paris Cedex 09 (FR)**

## Description

Technical field

This invention relates to a novel medicament for the prevention and the treatment of liver cirrhosis. More particularly, this invention relates to a medicament for the prevention and the treatment of liver cirrhosis, which comprises, as the principal component, the alcohol extract of fruits of wild grapes.

Background art

Liver cirrhosis is a kind of disease wherein a large amount of collagen fibres are formed in the liver due to various causes such as alcohol, virus, self immunity, and the like, resulting in the necrosis and function loss of the liver cells, and the patient will finally die. Liver cirrhosis frequently results in the production of liver cancer as well.

In spite of earnest efforts of many people, however, there has not as yet been found any effective medicament for the prevention and the treatment of liver cirrhosis. At best, the function loss of the liver cells has been slightly prevented.

An object of this invention is therefore to provide a novel medicament having a marked effect for the prevention and the treatment of liver cirrhosis.

Disclosure of invention

We have found as a result of an intensive study that the alcohol extract of fruits of wild grapes [scientific name: Ampelopsis Brevipedunculata (Maxim) Trautv.] is remarkably effective for the prevention and the treatment of liver cirrhosis. It has been already shown (Journal of the Taiwan Pharmaceutical Association, Vol. 19 No 1, p. 15—19 (1967)) that the alcohol extract of dried roots of the species Lancei (Li) found in Taiwan possesses antibiotic and diuretic activities.

Generally speaking, grapes belong to a family of Rhamnales order in Dicotyledones of Angiosperms. They are classified into twelve genuses and about 700 species. Among these grapes, five genuses and fourteen species have been found in Japan.

In this invention, only the fruits of wild grapes were selected and used. The wild grapes are a perennial plant of grape family and a creeper which may be commonly found in mountains and fields. They bloom in summer with small pale yellowish green flowers having five petals. After the bloom they bear global juicy fruits. The fruits ripen to a mixture of white, purple and green fruits.

In the use of the fruits of wild grapes following this invention, a bunch of grapes is thoroughly washed with water, followed by subjecting to extraction of any form, for example, an intact form, a crushed form, or a form wherein skin and seeds are excluded. The extraction agent in the present process is ethyl alcohol which may also be used in a form of a mixture with water, that is, an aqueous alcohol solution. Any mixing rate (rate of dilution) can be employed, and there are preferably used the rates ranging from 20% to 50%. There is no limitation in the amount of alcohol to be used for the extraction of the fruits. However about 1.5 times by weight or more of the alcohol to fruits is usually employed.

The extraction may be carried out at ordinary temperature, and usually requires from about six days to about six months. An especially long term is not always advantageous, and a sufficient extraction is usually achieved in a period of two months.

Brief description of drawing

Fig. 1 shows an infrared spectrum of the extract of fruits of wild grapes.

Fig. 2 shows a photomicrograph of M-cells cultured in the absence of the medicament for the prevention and the treatment of liver cirrhosis according to this invention.

Fig. 3 shows a photomicrograph of M-cells cultured in the presence of the medicament for the prevention and the treatment of liver cirrhosis according to this invention.

Best mode of carrying out the invention

This invention is illustrated practically by the following example, referring to the drawing and photomicrographs. However, this invention is not limited to this example.

Example

Fruits of wild grapes were thoroughly washed with water. About one kilogram of the fruits was dipped into 1.5 litres of about 40% aqueous ethyl alcohol solution at room temperature for about two months. The extract was distilled in an evaporator to remove the alcohol, and concentrated to produce a brown viscous liquid. Data of infrared spectrum analysis show that the product contains saccharides, peptides and organic acids.

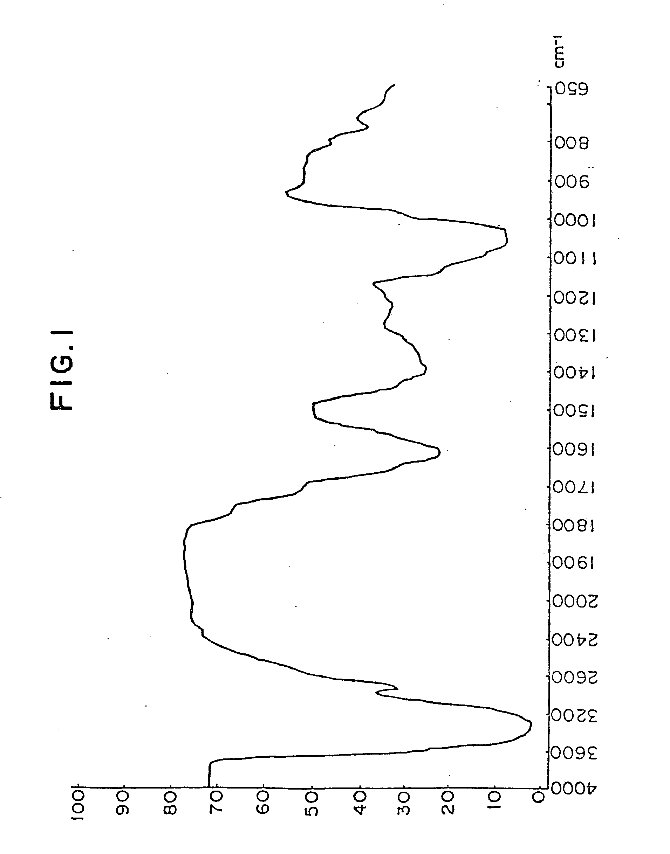

In Fig. 1, absorptions at about 1050 cm$^{-1}$ and at about 1400 cm$^{-1}$ show the presence of saccharides; absorptions at about 1610 cm$^{-1}$ and at about 3240 cm$^{-1}$ show the presence of peptides; and absorptions at about 1700 cm$^{-1}$ and at about 1750 cm$^{-1}$ show the presence of organic acids, respectively.

Experiment

Phenomena were traced in vitro by tissue culture method with medicament, that is, the alcohol extract of fruits of wild grapes according to this invention.

As the cells, there was used a transformed substrain of rat liver cells (hereinafter referred to M-cells) which was prepared by initiating the culture of February 4, 1963, the culture being derived from liver cells of a 10 days old P 16 rat of JAR-1 line, followed by subjecting to Nagisa culture and DAB-treatment. The term "DAB-treatment" used herein refers to the treatment wherein, on the basis of the information that liver cancer is generated when DAB, that is, 4-dimethylamino benzene (common name: Butter Yellow) is fed to rats, the cells originating from the rat liver are incubated for several days or more in a culture media containing DAB, to cause a substrain of the cells, resulting in liver cancer-like cells.

The substrain produces $\alpha$-fetoprotein, and is characterized by the production of a large amount of collagen fibres in the medium which seems to be undifferentiated cells of the liver. The substrain appears to be, so-called, liver cirrhosis cells.

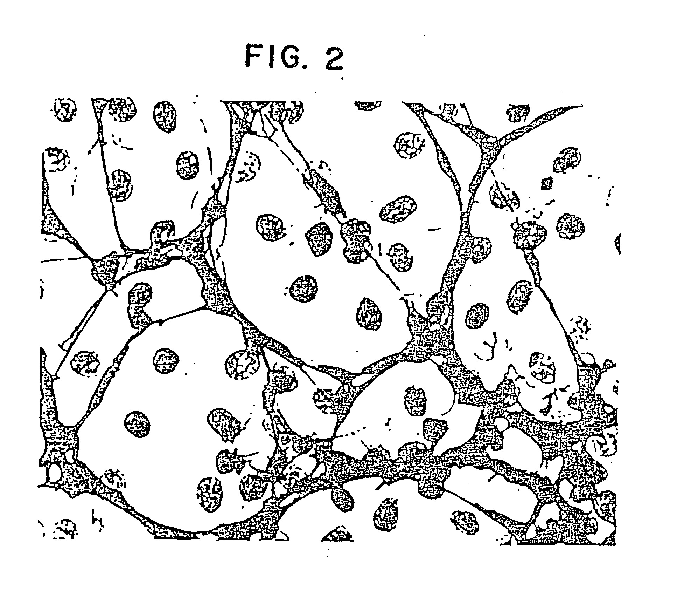

The extract of fruit of wild grapes was added to the culture of M-cells. The M-cells were cultivated without adding the extract of fruits of wild grapes, in culture tubes having flattened surfaces wherein a rectangular cover glass is placed. After a certain period of cultivation, the cells (control) were fixed and stained by argyophilic staining. Fig. 2 shows a photomicrograph (400 magnification) of the control culture of the cells after a week of cultivation.

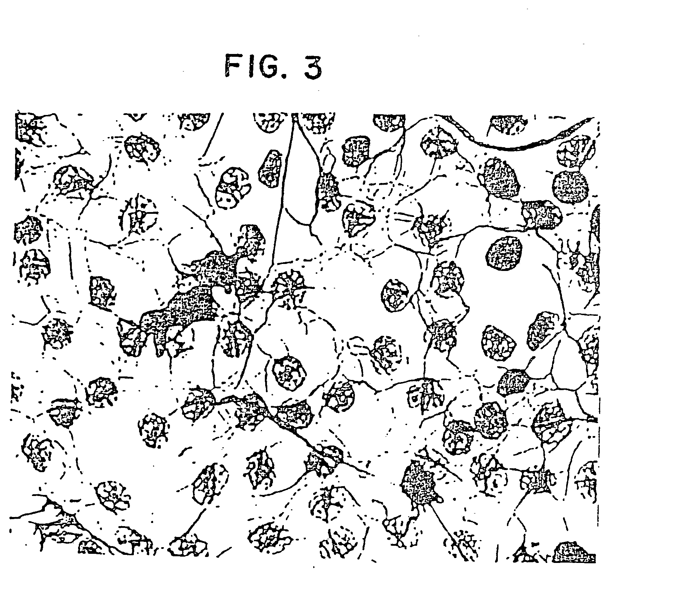

Fig. 3 shows a photomicrograph (400 magnification) of the cells in the case of an addition of the extracts of fruits of wild grapes, after a week of cultivation, which was obtained by the same procedures.

In Fig. 2, the formation of argyophilic fibers (collagen fibres) are abundantly observed, after a week of cultivation in the control wherein no addition of the extract of fruits of wild grapes is effected.

On the contrary, there was observed, in Fig. 3, only fine microfibres after a week of cultivation in the case wherein the extract of fruits of wild grapes was added.

It was thus confirmed that the extract of fruits of wild grapes according to this invention has a marked inhibitory effect on the formation of the collagen fibres of liver cells.

Industrial applicability

The medicament for the prevention and the treatment of the liver cirrhosis according to this invention may be orally administered in a liquid form of the extract of the fruits of wild grapes as it is, or in a dried powder or in a combination with any adjuvants or excipients.

The dose of the medicament for the prevention and the treatment of liver cirrhosis according to this invention varies from about 0.1 to about 1 g/Kg/day as an alcoholic extract.

## Claims

1. A medicament for the prevention and the treatment of liver cirrhosis in an orally administrable form, in particular in liquid form, which comprises the ethyl alcohol or aqueous ethyl alcohol extract of the fruits of the wild grapes.

2. The medicament for the prevention and the treatment of liver cirrhosis according to claim 1, wherein the whole fruit of wild grapes is extracted with ethyl alcohol.

3. The medicament for the prevention and the treatment of liver cirrhosis according to claim 1, wherein the crushed fruit of said wild grapes is extracted with ethyl alcohol.

4. The medicament for the prevention and the treatment of liver cirrhosis according to claim 1, wherein said fruit of wild grapes with skin and seeds removed, is extracted with ethyl alcohol.

5. The medicament for the prevention and the treatment of liver cirrhosis according to claim 1, wherein said ethyl alcohol is used in admixture with water.

6. The medicament for the prevention and the treatment of liver cirrhosis according to claim 1, wherein said ethyl alcohol is used in an amount of 1.5 times by weight or more of said fruit of wild grapes.

7. The medicament for the prevention and treatment of liver cirrhosis according to claim 1 wherein said extract has the infrared spectrum depicted in Figure 1.

8. The medicament for the prevention and treatment of liver cirrhosis according to claim 6 wherein the ethyl alcohol extraction is performed for between six days and six months.

9. The medicament for the prevention and treatment of liver cirrhosis according to claim 8 wherein the extraction is performed for at least about two months.

10. The medicament for the prevention and treatment of liver cirrhosis according to claim 6 wherein 1.5 liters 40% aqueous ethyl alcohol is employed to extract 1 kilogram of fruit for about two months.

## Patentansprüche

1. Medikament zur Verhütung und Behandlung der Leberzirrhose in oral verabreichbarer Form, insbesondere in flüssiger Form, enthaltend einen ethylalkoholischen oder wäßigethylalkoholischen Extrakt der Früchte des Ampelopsis brevipedunculata.

2. Medikament zur Verhütung und Behandlung der Leberzirrhose nach Anspruch 1, dadurch gekennzeichnet, daß die ganzen Früchte des Ampelopsis brevipedunculata mit Ethylalkohol extrahiert werden.

3. Medikament zur Verhütung und Behandlung der Leberzirrhose nach Anspruch 1, dadurch gekennzeichnet, daß die zerkleinerten Früchte des Ampelopsis brevipedunculata mit Ethylalkohol extrahiert werden.

4. Medikament zur Verhütung und Behandlung der Leberzirrhose nach Anspruch 1, dadurch gekennzeichnet, daß die Früchte des Ampelopsis brevipedunculata nach dem Entfernen der Haut und der Samen mit Ethylalkohol extrahiert werden.

5. Medikament zur Verhütung und Behandlung der Leberzirrhose nach Anspruch 1, dadurch gekennzeichnet, daß der Ethylalkohol im Gemisch mit Wasser verwendet wird.

6. Medikament zur Verhütung und Behandlung der Leberzirrhose nach Anspruch 1, dadurch gekennzeichnet, daß der Ethylalkohol in einer Menge des anderthalbfachen Gewichts der Früchte des Ampelopsis brevipedunculata oder einer größeren Menge verwendet wird.

7. Medikament zur Verhütung und Behandlung der Leberzirrhose nach Anspruch 1, dadurch gekennzeichnet, daß der Extrakt das in der Figur 1 dargestellte Infrarotspektrum besitzt.

8. Medikament zur Verhütung und Behandlung der Leberzirrhose nach Anspruch 6, dadurch gekennzeichnet, daß die Ethylalkohol-Extraktion über eine Zeitspanne zwischen sechs Tagen und sechs Monaten durchgeführt wird.

9. Medikament zur Verhütung und Behandlung der Leberzirrhose nach Anspruch 8, dadurch gekennzeichnet, daß die Ethylalkohol-Extraktion über eine Zeitspanne von wenigstens zwei Monaten durchgeführt wird.

10. Medikament zur Verhütung und Behandlung der Leberzirrhose nach Anspruch 6, dadurch gekennzeichnet, daß 1,5 l 40-proz. wäßriger Ethylalkohol zur Extraktion von 1 kg der Früchte während einer Zeitdauer von etwa zwei Monaten eingesetzt wird.

**Revendications**

1. Médicament pour la prévention et le traitement de la cirrhose du foie sous une forme administrable par voie orale, en particulier sous forme liquide, qui comprend un extrait des fruits d'Ampelopsis brevipedunculata dans l'alcool éthylique ou dans l'alcool éthylique aqueux.

2. Médicament pour la prévention et le traitement de la cirrhose du foie selon la revendication 1, dans lequel la totalité du fruit d'Ampelopsis brevipedunculata est extraite avec de l'alcool éthylique.

3. Médicament pour la prévention et le traitement de la cirrhose du foie selon la revendication 1, dans lequel le fruit d'Ampelopsis brevipedunculata, écrasé, est extrait avec de l'alcool éthylique.

4. Médicament pour la prévention et le traitement de la cirrhose du foie selon la revendication 1, dans lequel le fruit d'Ampelopsis brevipedunculata dont on a séparé la peau et les pépins, est extrait avec le d'alcool éthylique.

5. Médicament pour la prévention et le traitement de la cirrhose du foie selon la revendication 1, dans lequel l'alcool éthylique est utilisé en mélange avec de l'eau.

6. Médicament pour la prévention et le traitement de la cirrhose du foie selon la revendication 1, dans lequel l'alcool éthylique est utilisé en une quantité de 1,5 fois ou plus du poids du fruit d'Ampelopsis brevipedunculata.

7. Médicament pour la prévention et le traitement de la cirrhose du foie selon la revendication 1, dans le quel l'extrait a le spectre infrarouge représenté sur la figure 1.

8. Médicament pour la prévention et le traitement de la cirrhose du foie selon la revendication 6, dans lequel l'extraction avec de l'alcool éthylique est effectuée pendant de 6 jours à 6 mois.

9. Médicament pour la prévention et le traitement de la cirrhose du foie selon la revendication 8, dans lequel l'extraction est effectuée pendant au moins environ 2 mois.

10. Médicament pour la prévention et le traitement de la cirrhose du foie selon la revendication 6, dans lequel on utilise 1,5 litre d'alcool éthylique aqueux à 40% pour extraire 1 kilogramme de fruits pendant environ 2 mois.

FIG.1

## FIG. 2

## FIG. 3